# EUROPÄISCHE PATENTSCHRIFT

(11) **EP 1 419 391 B1**
(45) Veröffentlichungstag und Bekanntmachung des Hinweises auf die Patenterteilung: **21.09.2011**
(21) Anmeldenummer: 02758148.7
(22) Anmeldetag: 14.08.2002
(51) Int. Cl.: G01N 33/543

(54) **TRÄGER FUR IMMOBILISIERTE BIOMOLEKÜLE UND VERFAHREN ZUR IMMOBILISIERUNG VON BIOMOLEKÜLEN**
CARRIER FOR IMMOBILIZED BIOMOLECULES AND METHOD FOR BIOMOLECULE IMMOBILIZATION
SUPPORT POUR BIOMOLECULES IMMOBILISEES ET PROCEDE POUR IMMOBILISER DES BIOMOLECULES

(30) Priorität: 16.08.2001 DE 10141387
(43) Veröffentlichungstag der Anmeldung: 19.05.2004
(73) Patentinhaber: pes Gesellschaft für medizinische Diagnosesysteme GmbH, 04416 Markkleeberg (DE)
(72) Erfinder: KATERKAMP, Andreas, D- 34212 Melsungen (DE); MEYER, Ulrich, D-55270 Sörgenloch (DE); RAUCH, Peter, 48301 Nottuln (DE); ZELLMER, Angela, 59394 Nordkirchen (DE); WEBER, Silke, 31749 Auetal (DE); WOLF, Inka, D- 49767 Twist (DE); KEY, Göran, 49084 Osnabrück (DE); MEUSEL, Markus, 48159 Münster (DE); HAALCK, Lutz, 48149 Münster (DE); POHL, Stefan, D- 54470 Bernkastel-Kues (DE)
(74) Vertreter: Carlsohn, Alexander
(86) Internationale Anmeldenummer: PCT/DE2002/003031
(87) Internationale Veröffentlichungsnummer: WO 2003/016911

(56) Entgegenhaltungen:
- WO-A-95/24631
- WO-A-96/18498
- WO-A-96/22533
- DEVINE P L ET AL: "Monoclonal antibodies reacting with the MUC2 mucin core protein." BRITISH JOURNAL OF CANCER. ENGLAND JUN 1993, Bd. 67, Nr. 6, Juni 1993 (1993-06), Seiten 1182-1188, XP009003823 ISSN: 0007-0920

## Beschreibung

Die Erfindung betrifft Träger für immobilisierte Biomoleküle sowie ein Verfahren zur Immobilisierung solcher Biomoleküle auf Oberflächen von Trägern, die für die Durchführung von Affinitätsassays, wie z.B. Immunoassays, Rezeptor-Ligand, Nukleinsäure-Protein, Nukleinsäure-Nukleinsäure, Zucker-Lectin, anwendbar sind. Mit der Erfindung können die unterschiedlichen an sich bekannten Assayformate, wie z.B. Sandwich-Assays, durchgeführt werden. Die erfindungsgemäße Lösung kann auch vorteilhaft für Fluoreszenzimmunoassays eingesetzt werden.

Insbesondere für solche Fluoreszenzimmunoassays ist es vorteilhaft und gewünscht solche Biomoleküle, die häufig Proteine sind, in ausreichender Anzahl vor der eigentlichen Durchführung der Analysen unmittelbar auf Oberflächen von Trägern zu immobilisieren, um quantitativ bestimmte Analyte in einer Probe detektieren zu können.

Für die kovalente und adsorptive Immobilisierung auf Kunststoffoberflächen wurden bisher unterschiedliche Vorbereitungsarbeiten durchgeführt und z.B. Kunststoffoberflächen in der Regel chemisch modifiziert, wobei unter anderem sekundäre Amine oder Bromacetyl benutzt oder eine Chlorsulfonierung durchgeführt worden ist. Damit ist aber eine sehr aufwendige Präparation der Kunststoffoberflächen verbunden und für einige Kunststoffarten führt dieser Weg auch nicht zum Erfolg.

Devine P L et al., Br. J. Cancer (1993), 67, 1182-1188, offenbaren ein Verfahren zur Immobilisierung von Biomolekülen, bei dem die Biomoleküle auf einer Assayplatte in einem Carbonatpuffer bei 4 °C inkubiert werden, sowie eine alternatives Verfahren, bei dem die Biomoleküle durch Trocknen des Peptides auf der Assayplatte immobilisiert werden.

Es ist daher Aufgabe der Erfindung, die Immobilisierung von Biomolekülen auf Trägern zu vereinfachen und zu verbessern.

Erfindungsgemäß wird diese Aufgabe mit einem Träger, der die Merkmale des Anspruchs 1 aufweist und einem Verfahren gemäß Anspruch 15 gelöst. Vorteilhafte Ausgestaltungsformen und Weiterbildungen der Erfindung können mit den in den untergeordneten Ansprüchen enthaltenen Merkmalen erreicht werden.

Der erfindungsgemäße Träger kann aus den verschiedensten Materialien, wie Glas, Metall, anorganischen Verbindungen, verschiedensten Kunststoffen und insbesondere aus Polymethylmethacrylat (PMMA), Polycarbonat, Polystyrol, Cycloolefinpolymere und -copolymere, Polyethylen, Polypropylen in optischer Qualität gebildet sein. Auf der Oberfläche, auf der die jeweiligen Biomoleküle immobilisiert sind, sind diese Biomoleküle in einer Substanz mit ionischem Charakter oder einem Gemisch, das aus mindestens zwei unter schiedlichen solcher Substanzen besteht, eingebettet,

wobei zumindest ein Teil der Biomoleküle auf der entsprechenden Oberfläche des Trägers immobilisiert ist. Die Substanz(en) bildet/bilden eine Kruste auf dem Träger.

Die Immobilisierung dieser Biomoleküle auf der Oberfläche eines Trägers wird so vorgenommen, dass eine Pufferlösung, in der die Biomoleküle enthalten sind, mit hoher Konzentration an einer oder mehreren Substanzen mit ionischem Charakter auf die Oberfläche des Trägers aufgebracht wird. Substanzen mit ionischem Charakter können Salze, wie z.B. Natriumchlorid oder Amoniumsulfat, Polyanionen, wie z.B. Polycarbonsäure, Polykationen, wie z.B. Polyethylenemin, Zwitterionen, wie z.B. Betain oder Cholin, Ampholyte, wie z.B. isomere aliphatischer Oligoamino-Oligocarbonsäuren und Salze mit denen eine Pufferlösung hergestellt wird, sein. So sind die Salze der Hofmeisterserie geeignet. Die Konzentration des Salzes sollte im Bereich zwischen 100 mM bis 1,5 M liegen. Nach dem Aufbringen der so vorbereiteten Pufferlösung wird das Lösungsmittel, in der Regel entionisiertes Wasser aber auch organische Lösungsmittel bzw. Lösungsmittelgemische, durch Trocknung entfernt, dabei die Biomoleküle in die Substanz(en) eingebettet und zumindest ein Teil der Biomoleküle auf der Oberfläche des Trägers immobilisiert.

Ein so vorbereiteter Träger kann dann einer Probe ausgesetzt und für die jeweilige Probe ein Affinitätsassay durchgeführt werden.

Da die flüssige Probe bereits bei Beginn der Durchführung von Affinitätsassays mit den Substanzen in Kontakt treten kann, wird die eine oder mehrere Substanzen zumindest teilweise in der Probe gelöst und bei bestimmten Verfahren zur Durchführung von Affinitätsassays, bei denen die jeweilige flüssige Probe während der Durchführung der Assays über die auf einer Oberfläche immobilisierten Biomoleküle entlang strömt, die gelöste Substanzen aus dem eigentlichen Detektionsbereich abgeführt, so dass die Detektion geringfügig zeitversetzt durchgeführt werden sollte.

Mit der Erfindung können unterschiedlichen Biomoleküle, wie Nukleinsäuren, (Glyko-)Proteine, (Glyko)Peptide, (Glyko-)Lipide oder Kohlenhydrate und so z.B. Antikörper und/oder Neutravidin, aber auch Avidin, Albergene CRP, Hämoglobin, hCG, hFabp, IgE oder Myoglobin eingesetzt werden, wobei auch mehrere unterschiedliche Biomoleküle auf einer Oberfläche immobilisiert werden können. Desweiteren können auch niedermolekulare Verbindungen, die an solche Biomoleküle gebunden sind, so auf Oberflächen immobilisiert werden.

Als bevorzugt einzusetzende Salze, die der Pufferlösung zugegeben werden können, haben sich Natriumchlorid und Ammoniumsulfat herausgestellt, wobei Konzentrationen zwischen 150 mM und 1 M insbesondere für Natriumchlorid und Konzentrationen zwischen 300 und 800 mM insbesondere für Ammoniumsulfat besonders zu bevorzugen sind.

Als Pufferlösungen können die verschiedensten an sich bekannten, wie z.B. Phosphat-, Carbonat-, Citrat-, Acetat-, Tris-, Triethanolamin-, Hepes-, Pipes- oder Mopspuffer, um nur einige Beispiele zu nennen, eingesetzt werden. Dabei sollte die Auswahl des jeweiligen Puffers insbesondere unter Berücksichtigung der jeweils zu immobilisierenden Biomoleküle vorgenommen werden.

So hat sich beispielsweise für zu immobilisierende Antikörper eine Phosphatpufferlösung mit Natriumchlorid als vorteilhaft herausgestellt. Neben der Auswahl einer biomolekülspezifischen Pufferlösung sollte auch ein biomolekülspezifischer pH-Wert eingehalten werden. Der pH-Wert sollte generell im Bereich zwischen 3 bis 10 gehalten werden.

So kann beispielsweise bei einer Phosphatpufferlösung durch entsprechende Anteile an Na₂HPO₄ und KH₂PO₄ ein pH-Wert im Bereich zwischen 5 bis 8 eingestellt werden.

Es hat sich insbesondere für eine längere Lagerungsperiode von entsprechend vorbereiteten Trägern herausgestellt, dass es vorteilhaft ist, der Pufferlösung Stabilisatoren, z.B. Kohlenhydrate zuzugeben, die dann ebenfalls neben den Biomolekülen in den Substanzen bzw. Substanzgemisch auf der Oberfläche enthalten sind. So kann vorteilhaft als ein Kohlenhydrat Sorbit eingesetzt werden.

Für einige Trägermaterialien, wie z.B. Kunststoffe, kann es ebenfalls vorteilhaft sein, die Oberfläche des Trägers, auf der die Biomoleküle immobilisiert werden sollen, vorab physiko-chemisch zu modifizieren.

Durch eine solche Modifizierung kann dann auch die Grenzflächenspannung der modifizierten Trägeroberfläche reduziert werden.

Diese Reduzierung kann durch eine Verringerung des Randwinkels eines liegenden entionisierten Wassertropfens auf der modifizierten Trägeroberfläche um mindestens 5°, bevorzugt um mindestes 10° quantifiziert werden.

Dabei sollte der fort- und zurückschreitende Kontaktwinkel, mit einem Messgerät der Firma Krüss mit der Typenbezeichnung G-1 dynamisch bestimmt und durch die Modifizierung der Randwinkel eines fortschreitenden Tropfens in den Bereich zwischen 45° bis 55° und eines rückschreitenden Tropfens in den Bereich zwischen 30 bis 40°, bei einem Träger aus PMMA reduziert werden.

Da die Substanzen ionischen Charakters, in denen die Biomoleküle auf der Trägeroberfläche eingebettet sind, hygroskopisch sind, sollten die entsprechend vorbereiteten Träger im Zeitraum zwischen der eigentlichen Immobilisierung und der Durchführung der Affinitätsmessung, der einige Monate betragen kann, in eine Umhüllung mit sehr geringer Wasserdampfdurchlässigkeit eingeschlossen werden, um eine Wasseraufnahme zu vermeiden. So kann beispielsweise eine solche Umhüllung aus einer randseitig verschweißten metallbeschichteten Kunststoff-Folie gebildet werden.

In eine solche Umhüllung kann zusätzlich ein feuchtigkeitsaufnehmendes und speicherndes Material eingeschlossen werden.

Die Biomoleküle und ggf. das zusätzliche Salz enthaltende Pufferlösung kann auf unterschiedliche Art und Weise auf die Trägeroberfläche aufgebracht werden. So kann die Pufferlösung auf die Oberfläche dispensiert werden, wobei die einzelnen Tropfen mit vorgebbaren Volumina im Bereich zwischen wenigen nl bis µl auf die Oberfläche lokal definiert abgelegt werden können. Es besteht aber auch die Möglichkeit eine sogenannte Tauchbeschichtung vorzunehmen, wobei der Träger möglichst mit definierten Geschwindigkeiten in die Pufferlösung eingetaucht und anschließend herausgezogen werden sollte. Es kann aber auch das sogenannte Spin-Coating eingesetzt werden.

Die ggf. die zusätzliche Substanz ionischem Charakter, die Biomoleküle und gegebenenfalls auch Kohlenhydrat enthaltende Pufferlösung bewirkt durch die hohe Konzentration der Substanz eine Beeinflussung der Biomoleküle, wobei diese aus einer wasserlöslichen in eine wasserunlösliche Form überführt werden. So wird eine höhere Affinität der Biomoleküle zur entsprechenden Kunststoffoberfläche bewirkt. Dadurch können die Biomoleküle nach der Entfernung des Lösungsmittels verbessert auf der Trägeroberfläche immobilisiert werden.

Die Trocknung zur Entfernung des Lösungsmittels kann unter verschiedenen Bedingungen durchgeführt werden, wobei bestimmte Einflüsse berücksichtigt werden sollten. So werden mit den unterschiedlichen Verfahren zur Aufbringung der Pufferlösung unterschiedliche Volumina und auch unterschiedliche Schichtdicken ausgebildet. Des weiteren spielen die Umgebungsbedingungen und hier insbesondere die Temperatur und Luftfeuchtigkeit eine Rolle. So können beispielsweise die Trocknung in einem Reinraum bei einer Temperatur von 20 °C und einer relativen Luftfeuchtigkeit von 40 bis 50 % durchgeführt werden. Es kann aber auch eine Vakuumtrocknung bei 50 °C oder bei erhöhten Temperaturen durchgeführt werden.

Im Anschluss an die Trocknung und Entfernung von Lösungsmittel kann eine zusätzliche stabilisierende Temperung mit Erwärmung durchgeführt werden.

Die bereits erwähnte Modifizierung der Trägeroberfläche, auf der die Biomoleküle immobilisiert werden sollen, kann auf verschiedene Art und Weise durchgeführt werden. So besteht die Möglichkeit, die Oberflächenänderung physiko-chemisch durch thermische, plasmachemische, elektrochemische oder nasschemische Prozesse durchgeführt werden.

So kann die entsprechende Oberfläche einer Korona- oder Barriereentladung ausgesetzt werden. Eine weitere Möglichkeit besteht darin, ein Niederdruck- oder Hochdruckplasma auf die Oberfläche einwirken zu lassen, wobei das jeweilige Plasma vorteilhaft mit Sauerstoff, Kohlendioxid oder Stickstoff erzeugt wird. Es können aber auch andere Gase für die Plasmaerzeugung eingesetzt werden.

Die Oberflächenmodifizierung kann aber auch thermisch mit einem üblicherweise als Temperprozess bezeichneten Verfahren durchgeführt werden. Dabei kann der Träger gezielt erwärmt werden, wobei eine Mindesttemperatur von 40 °C erreicht werden sollte. Diese Temperatur kann dann über einen bestimmten Zeitraum gehalten und sich dann eine Abkühlphase mit definierter Abkühlrate durchgeführt werden.

Soll die Modifizierung nasschemisch durchgeführt werden, hat es sich herausgestellt, dass die Oberfläche Kaliumpermanganat enthaltender Schwefelsäure ausgesetzt wird, wobei im Anschluß an diese Modifizierung ein ausreichendes Spülen durchgeführt werden sollte.

Die erfindungsgemäßen Träger können beispielsweise bei einer Vorrichtung und für die Durchführung eines Verfahrens, wie sie in DE 196 28 002 C1 beschrieben sind, eingesetzt werden, wobei in diesem Fall der Träger den Boden eines küvettenförmigen Flußkanals bilden kann.

Eine weitere Möglichkeit zur Ausgestaltung eines solchen Trägers ist die, wie sie in DE 197 47 572 C1 beschrieben ist und dabei die Biomoleküle auf der äußeren Fläche eines Lichtwellenleiters immobilisiert werden.

Vorteilhaft ist es, wenn der Anteil der auf der Oberfläche des Trägers gebildeten kubischen Kristalle nicht größer als 50 Vol.-%, und besonders vorteilhaft nicht größer als 30 Vol.-% ist.

Die ionische Substanz sowie die Biomoleküle sind auf dem Träger nicht kovalent gebunden.

Nachfolgend sollen einige Beispiele für die erfindungsgemäße Lösung erläutert werden.

So hat es sich herausgestellt, dass beispielsweise für die Immobilisierung von Antikörpern Phosphatpufferlösungen mit zusätzlichem Natriumchlorid besonders geeignet sind.

So kann eine solche Phosphatpufferlösung durch entsprechende Konzentrationen von Na₂HPO₄ und KH₂PO₄ einen pH-Wert zwischen 6,8 und 7,2 eingestellt werden und in der Phosphatpufferlösung 600 mM Natriumchlorid enthalten sein. Die Antikörperkonzentration in der Phosphatpufferlösung kann dann bei 0,5 mg/ml liegen.

So vorbereitete Phosphatpufferlösung wurde mit einem Dispenser auf eine PMMA-Oberfläche gegeben und pro Tropfen ca. 20 nl aufgebracht.

Nach dem Dispensieren wurde eine Trocknung bei einer Luftfeuchtigkeit zwischen 35 bis 40 % durchgeführt. Dadurch konnte das Lösungsmittel Wasser entfernt und die Antikörper in die auf der Oberfläche ausgebildeten Salzkrusten eingebettet und ein großer Teil unmittelbar auf der Kunststoffoberfläche adsorptiv immobilisiert werden. Dadurch konnte ein deutlich höhere funktionelle Belegungsdichte, die auch homogen verteilt ist, erreicht werden, so dass der Detektionsbereich für die Immunoassays erweitert werden kann.

Es kann aber eine Phosphatpufferlösung mit 300 mM Ammoniumsulfat, deren pH-Wert im Bereich zwischen 5 und 8 liegt, eingesetzt werden. Einer solchen Phosphatpufferlösung kann außerdem 0,1 bis 1 % Sorbit bzw. ein anderes Kohlenhydrat zugegeben werden, um eine zusätzliche stabilisierende Wirkung für die Biomoleküle zu erreichen.

Anstelle der Phosphatpufferlösungen können neben den anderen bereits vorab erwähnten Pufferlösungen auch günstigerweise für bestimmte Biomoleküle Carbonatpufferlösungen auch mit einem zusätzlich enthaltenen Salz eingesetzt werden, wobei jedoch bei solchen Carbonatpufferlösungen der pH-Wert größer ist, und im Bereich zwischen 9 und 10 liegt. Carbonatpufferlösungen lassen sich in Verbindung mit Natriumchlorid besonders günstig für die Immobilisierung von Avidin, Albergene, CRP, Hämoglobin, hCG, hFabp, IgE oder Myoglobin einsetzen.

Die Modifizierung der Oberfläche eines PMMA-Trägers kann beispielsweise mit einer Koronaentladung erreicht werden. Im Anschluß an die Koronabehandlung kann die Oberfläche mit ionisierter Druckluft abgeblasen und dann die Pufferlösung, wie bereits beispielhaft beschrieben, auf die entsprechend modifizierte Oberfläche aufgebracht werden.

Nach Trocknung und Entfernung des Lösungsmittels sollte die Lagerung bei Luftfeuchtigkeiten nicht größer 10 % und bei entsprechend niedrigeren Temperaturen unter 10 °C durchgeführt werden.

Soll eine thermische Modifizierung der Oberfläche eines Trägers durchgeführt werden, kann ein Träger aus PMMA auf eine Temperatur von 85 °C über einen Zeitraum von mehreren Stunden erwärmt werden. Danach wird der erwärmte Träger mit einer Abkühlrate von 10 °C/h auf eine Temperatur von 45 °C gekühlt und anschließend mit Stickstoff beblasen.

Der so modifizierte Träger kann dann in eine Phosphatpufferlösung, in der 5 mg/ml Neutravidin in 5 mM Phosphatpufferlösung bei einem pH-Wert von 7,4 und zusätzlich 300 mM Ammoniumsulfat enthalten sind, eingetaucht werden. Das Eintauchen und Herausziehen sollte mit einer Geschwindigkeit von jeweils 5 bis 10 mm/s, bevorzugt mit 8 mm/s erfolgen. Nach dem Herausziehen wird der Träger im Vakuum bei ca. 350 mbar und einer Temperatur von 50 °C innerhalb eines Zeitraums von 10 min getrocknet und das Wasser aus der Phosphatpufferlösung entfernt.

Der so vorbereitete Träger, an dem das Neutravidin als Biomoleküle immobilisiert worden ist, kann dann sofort für die Durchführung von Immunoassays genutzt oder wie bereits mehrfach erwähnt, trocken und Wasserdampf undurchlässig eingehüllt, gelagert und transportiert und erst später für die Durchführung der Assays genutzt werden.

Nachfolgend soll die Erfindung beispielhaft mit Figuren erläutert werden.

Dabei zeigen:
- Figur 1: ein Beispiel der Belegungsdichte mit Pro- teinen, sichtbar gemacht mit einem Partike- lassay. A herkömmliches und B erfindungsge- mäßes Immobilisierungsverfahren.
- Figur 2: einen Kristallbildungsnachweis mittels Weisslichtinterferometrie und
- Figur 3: Beispiele eines Lichtwellenleiters mit herkömmlich und erfindungsgemäß immobili- sierten Biomolekülen.

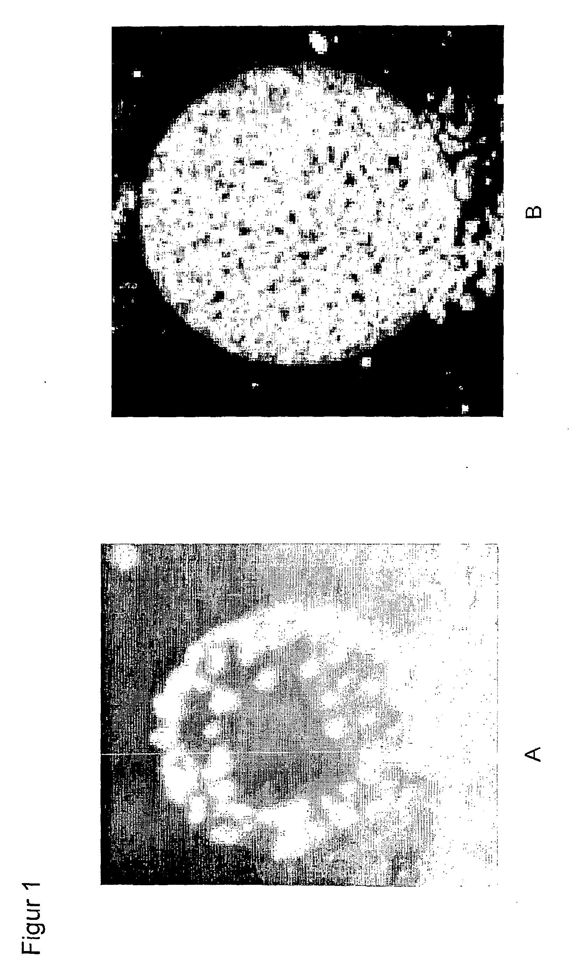

In der Figur 1 A ist die Proteinbelegung mit einem herkömmlichen Verfahren gezeigt. Die Proteine wurden durch eine partikelmarkierte spezifische Immunreaktion sichtbar gemacht, die auch die Belegungsdichte sichtbar macht.

Bei Figur 1 B wurden Antikörper mit einer Konzentration von 0,5 mg/ml in 10 mM Phosphatpufferlösung mit 600 mM NaCl, als Tropfen mit einem Volumen von 20 nl für die Immobilisierung verwendet.

Die Belegungsdichte und homogene Verteilung gegenüber der in Figur 1 B gezeigten herkömmlichen Lösung ist eindeutig erkennbar.

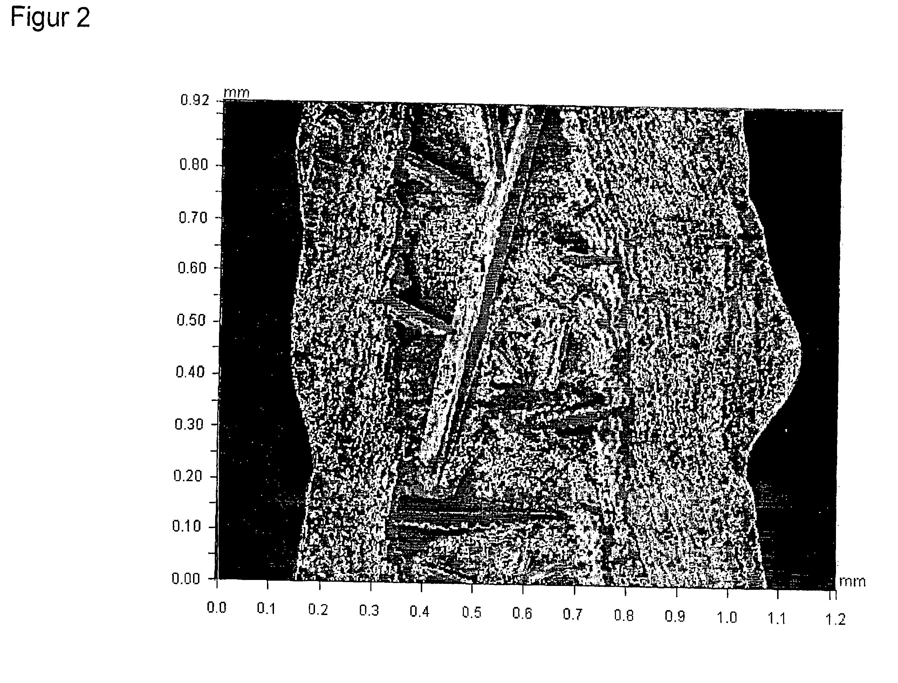

In Figur 2 ist ein Ausschnitt einer Linie mit erfindungsgemäß immobilisierten Biomolekülen auf einer Trägeroberfläche gezeigt. Durch die relativ hohe Konzentration des für die Immobilisierung verwendeten Natriumchlorides kann durch die Kristallbildung auf der Oberfläche ein mikroskopischer Nachweis oder ein Nachweis mit einem Weisslichtinterferometer geführt werden.

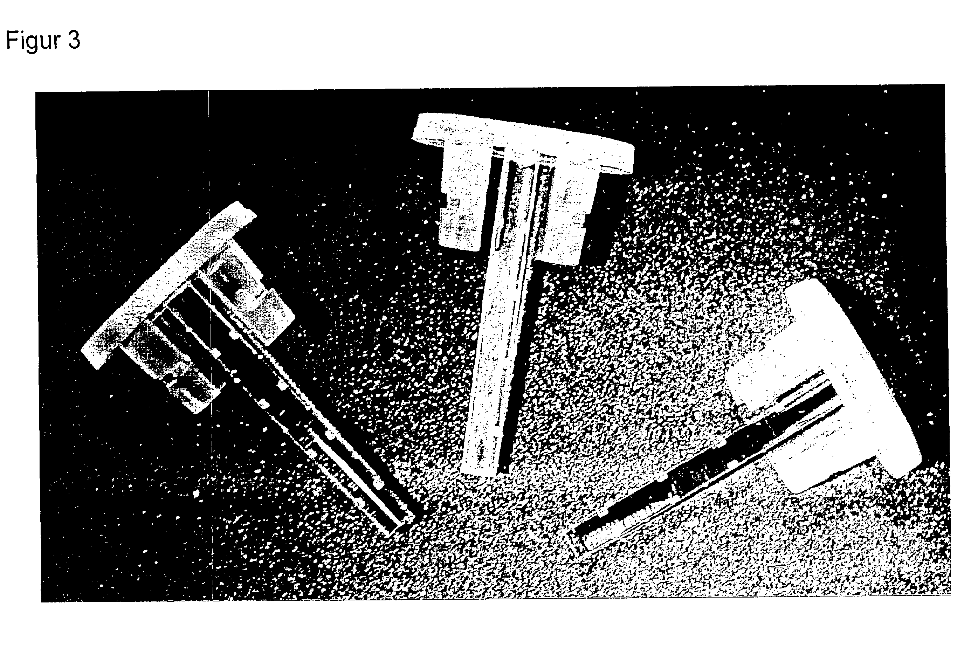

In Figur 3 sind Lichtwellenleiter gezeigt, wie sie in einer Vorrichtung, die in DE 197 47 572 C1 beschrieben ist, eingesetzt werden können. Dabei wurde bei dem in der Mitte angeordnete Lichtwellenleiter eine erfindungsgemäße Immobilisierung mit einer Phosphatpufferlösung und zusätzlich Ammoniumsulfat durchgeführt und bei den beiden äußeren die Immobilisierung herkömmlich durchgeführt.n Deutlich ist die Salzkruste auf dem mittleren Lichtwellenleiter erkennbar.

## Patentansprüche

1. Träger für immobilisierte Biomoleküle zur Durchführung von Affinitätsassays, bei dem auf einer Oberfläche des Trägers die Biomoleküle in einer Substanz mit ionischem Charakter aus der Gruppe der Salze, Polyanionen, Polykationen, Zwitterionen, Ampholyte und Salzen für Pufferlösungen oder einem Gemisch dieser Substanzen eingebettet und zumindest ein Teil der Biomoleküle auf der Trägeroberfläche immobilisiert sind.

2. Träger nach Anspruch 1, **dadurch gekennzeichnet, dass** ein Salz, in dem die Biomoleküle eingebettet sind, ein Salz aus der Hofmeisterserie ist.

3. Träger nach Anspruch 1 oder 2, **dadurch gekennzeichnet, dass** ein Salz, in dem die Biomoleküle eingebettet sind, Natriumchlorid oder Ammoniumsulfat ist.

4. Träger nach einem der Ansprüche 1 bis 3, **dadurch gekennzeichnet, dass** die Substanz(en) Salze für Pufferlösungen für Phosphat-, Carbonat-, Citrat-, Acetat-, Tris-, Triethanolamin-, Herpes-, Pipes-, oder Mopspuffer sind.

5. Träger nach einem der Ansprüche 1 bis 4, **dadurch gekennzeichnet, dass** in der Substanz oder dem Substanzgemisch zusätzlich mindestens ein Stabilisator für Biomoleküle enthalten ist.

6. Träger nach einem der Ansprüche 1 bis 4, **dadurch gekennzeichnet, dass** die Biomoleküle Nukleinsäuren, (Glyko-) Proteine, (Glyko-) Peptide, (Glylco-) Lipide oder Kohlenhydrate sind.

7. Träger nach einem der Ansprüche 1 bis 6, **dadurch gekennzeichnet, dass** die Biomoleküle Antikörper, Allergene, Avidin, CRP, Hämoglobin, hCG, hFabp, IgA, IgE, Myoglobin und/oder Neutravidin sind.

8. Träger nach einem der Ansprüche 1 bis 7, **dadurch gekennzeichnet, dass** der Träger für Lagerung und Transport trocken in eine nahezu wasserdampfundurchlässige Umhüllung eingeschlossen ist.

9. Träger nach einem der Ansprüche 1 bis 8, **dadurch gekennzeichnet, dass** das Trägermaterial ein Kunststoff, ein Glas, ein Metall oder eine anorganische Verbindung ist.

10. Träger nach einem der Ansprüche 1 bis 9, **dadurch gekennzeichnet, dass** der Träger aus Polymethylmethacrylat, Cycloolefinpolymere und -copolymere, Polyethylen, Polystyrol oder Polycarbonat gebildet ist.

11. Träger nach einem der Ansprüche 1 bis 10, **dadurch gekennzeichnet, dass** die Oberfläche des Trägers durch physiko-chemische Prozesse modifiziert ist.

12. Träger nach Anspruch 11, **dadurch gekennzeichnet, dass** die Oberfläche des Trägers infolge der Modifizierung eine reduzierte Grenzflächenspannung zu entionisicrtem Wasser aufweist.

13. Träger nach Anspruch 11, **dadurch gekennzeichnet, dass** infolge der Modifizierung der Kontaktwinkel eines entionisierten Wassertropfens um mindestens 5° gegenüber einer nicht modifizierten Oberfläche verringert ist.

14. Träger nach mindestens einem der Ansprüche 1 bis 13, **dadurch gekennzeichnet, dass** der Anteil an auf der Oberfläche des Trägers gebildeten kubischen Kristallen ≤ 50 Vol. -% ist.

15. Verfahren zur Immobilisierung von Biomolekülen auf Oberflächen von Trägern, für die Durchführung von Affinitätsassays, bei dem auf die Oberfläche des Trägers Biomoleküle in einer Pufferlösung, die mindestens eine Substanz mit ionischem Charakter aus der Gruppe der Salze, Polyanionen, Polykationen, Zwitterionen, Ampholyte und Salzen für Pufferlösungen enthält, aufgebracht, unmittelbar im Anschluss daran das Lösungsmittel aus der Pufferlösung durch Trocknung entfernt, dabei die Biomoleküle in die Substanz oder ein Gemisch dieser Substanzen eingebettet und zumindest ein Teil der Biomoleküle auf der Oberfläche des Trägers immobilisiert werden.

16. Verfahren nach Anspruch 15, **dadurch gekennzeichnet, dass** eine Pufferlösung mit einer Salzkonzentration im Bereich 100 mM bis 1,5 M verwendet wird.

17. Verfahren nach Anspruch 15 oder 16, **dadurch gekennzeichnet, dass** eine biologische Pufferlösung, die zusätzlich Stabilisatoren für die Biomoleküle enthält, verwendet wird.

18. Verfahren nach einem der Ansprüche 15 bis 17, **dadurch gekennzeichnet, dass** eine Pufferlösung, mit einem pH-Wert zwischen 3 und 10 verwendet wird.

19. Verfahren nach einem der Ansprüche 15 bis 18, **dadurch gekennzeichnet, dass** die jeweilige Oberfläche des Trägers vor dem Aufbringen der Biomleküle enthaltenden biologischen Pufferlösung physiko-chemisch durch thermische, plasmachemische, elektrochemische oder nasschemische Prozesse modifiziert wird.

20. Verfahren nach Anspruch 19, **dadurch gekennzeichnet dass** durch die Modifizierung die Grenzflächenspannung der Oberfläche des Trägers reduziert wird.

21. Verfahren nach Anspruch 19 oder 20, **dadurch gekennzeichnet, dass** die Modifizierung mit einer Korona- oder Barriereentladung durchgeführt wird.

22. Verfahren nach Anspruch 19, **dadurch gekennzeichnet, dass** eine Temperaturbehandlung mit einer Erwärmung des Trägers auf eine Temperatur von mindestens 40 °C und anschließend eine definierte Abkühlung durchgeführt wird.

23. Verfahren nach Anspruch 19, **dadurch gekennzeichnet, dass** eine nasschemische Behandlung mit Kaliumpermanganat enthaltender Schwefelsäure durchgeführt wird.

24. Verfahren nach Anspruch 19, **dadurch gekennzeichnet, dass** die Oberflächenmodifizierung mittels einer Hoch- oder Niederdruckgasplasmabehandlung durchgeführt wird.

25. Verfahren nach Anspruch 24, **dadurch gekennzeichnet, dass** das Gasplasma mit Sauerstoff, Stickstoff und/oder Kohlendioxid erzeugt wird.

26. Verfahren nach einem der Ansprüche 15 bis 25, **dadurch gekennzeichnet, dass** das Aufbringen mittels Dispensieren, Tauchbeschichten oder Spin-Coating erfolgt.

27. Verfahren nach einem der Ansprüche 15 bis 26, **dadurch gekennzeichnet, dass** die Trocknung zur Entfernung des Lösungsmittels im Vakuum und bei einer Temperatur von 50 °C innerhalb eines Zeitraums, der ca. 10 min nicht überschreitet, durchgeführt wird.

28. Verfahren nach einem der Ansprüche 15 bis 27, **dadurch gekennzeichnet, dass** die Trocknung zur Entfernung des Lösungsmittels bei Raumtemperatur und einer Luftfeuchtigkeit im Bereich zwischen 35 bis 40 % durchgeführt wird.

29. Verfahren nach einem der Ansprüche 15 bis 28, **dadurch gekennzeichnet, dass** der Träger während der Lagerung und Transport trocken mit einer für Wasserdampf nahezu undurchlässigen Umhüllung vollständig umschlossen wird.

## Claims

1. A carrier for immobilized biomolecules to perform affinity assays wherein on one surface of the carrier the biomolecules are embedded in a substance with ionic character from the group of the salts, polyanions, polycations, zwitterions, ampholytes, and salts for buffer solutions or in a mixture of said substances, and at least a part of the biomolecules is immobilized on the carrier surface.

2. The carrier according to claim 1, **characterized in that** a salt in which the biomolecules are embedded is a salt of the Hofmeister series.

3. The carrier according to claim 1 or 2, **characterized in that** a salt in which the biomolecules are embedded is sodium chloride or ammonium sulfate.

4. The carrier according to any one of claims 1 to 3, **characterized in that** the substance(s) are salts for buffer solutions for phosphate, carbonate, citrate, acetate, Tris, triethanolamine, HEPES, PIPES, or MOPS buffer.

5. The carrier according to any one of claims 1 to 4, **characterized in that** in the substance or the mixture of substances there is additionally contained at least one stabilizing agent for biomolecules.

6. The carrier according to any one of claims 1 to 4, **characterized in that** the biomolecules are nucleic acids, (glyco) proteins, (glyco) peptides, (glyco) lipids, or carbohydrates.

7. The carrier according to any one of claims 1 to 6, **characterized in that** the biomolecules are antibodies, allergens, avidin, CRP, haemoglobin, hCG, hFabp, IgA, IgE, myoglobin, and/or neutravidin.

8. The carrier according to any one of claims 1 to 7, **characterized in that** for storage and transport the carrier is dry enclosed in an almost moisture-proof envelope.

9. The carrier according to any one of claims 1 to 8, **characterized in that** the carrier material is a plastic, a glass, a metal, or an inorganic compound.

10. The carrier according to any one of claims 1 to 9, **characterized in that** the carrier is made of polymethyl methacrylate, cyclic olefin polymers and copolymers, polyethylene, polystyrene, or polycarbonate.

11. The carrier according to any one of claims 1 to 10, **characterized in that** the surface of the carrier is modified by physico-chemical processes.

12. The carrier according to claim 11, **characterized in that** due to the modification the surface of the carrier has a reduced interfacial tension to deionized water.

13. The carrier according to claim 11, **characterized in that** due to the modification the contact angle of a drop of deionized water is reduced by at least 5° over a not modified surface.

14. The carrier according to at least one of claims 1 to 13, **characterized in that** the portion of cubic crystals formed on the surface of the carrier is ≤ 50% by volume.

15. A method for immobilizing biomolecules on surfaces of carriers to perform affinity assays, wherein there are applied biomolecules in a buffer solution containing at least one substance with ionic character from the group of the salts, polyanions, polycations, zwitterions, ampholytes, and salts for buffer solutions onto the surface of the carrier, immediately afterwards the solvent is removed from the buffer solution by drying, at the same time the biomolecules are embedded in the substance or mixture of said substances, and at least a part of the biomolecules is immobilized on the surface of the carrier.

16. The method according to claim 15, **characterized in that** a buffer solution having a salt concentration in the range of 100 mM to 1.5 M is used.

17. The method according to claim 15 or 16, **characterized in that** a biological buffer solution additionally containing stabilizing agents for the biomolecules is used.

18. The method according to any one of claims 15 to 17, **characterized in that** a buffer solution with a pH value between 3 and 10 is used.

19. The method according to any one of claims 15 to 18, **characterized in that** the respective surface of the carrier is physico-chemically modified by thermal, plasma-chemical, electro-chemical, or wet-chemical processes before the application of the biological buffer solution containing biomolecules.

20. The method according to claim 19, **characterized in that** by the modification the interfacial tension of the surface of the carrier is reduced.

21. The method according to claim 19 or 20, **characterized in that** the modification is performed with a corona or barrier discharge.

22. The method according to claim 19, **characterized in that** a temperature treatment under heating the carrier to a temperature of at least 40°C and subsequently defined cooling is carried out.

23. The method according to claim 19, **characterized in that** a wet-chemical treatment with sulfuric acid containing potassium permanganate is carried out.

24. The method according to claim 19, **characterized in that** the surface modification is carried out by means of high or low-pressure gas plasma treatment.

25. The method according to claim 24, **characterized in that** the gas plasma is generated with oxygen, nitrogen, and/or carbon dioxide.

26. The method according to any one of claims 15 to 25, **characterized in that** the application is done by means of dispensing, dip coating, or spin coating.

27. The method according to any one of claims 15 to 26, **characterized in that** drying to remove the solvent is done in vacuum and at a temperature of 50°C within a period not exceeding ca. 10 min.

28. The method according to any one of claims 15 to 27, **characterized in that** drying to remove the solvent is done at room temperature and an air humidity in a range between 35 and 40%.

29. The method according to any one of claims 15 to 28, **characterized in that** the carrier is completely dry enclosed with an almost moisture-proof envelope during storage and transport.

## Revendications

1. Support pour biomolécules immobilisées pour exécuter des tests d'affinité dans lesquels à la surface du support, les biomolécules sont placées dans une substance à caractère ionique du groupe des sels, des polyanions, des polycations, des zwitterions, des ampholytes et des sels pour solutions tamponnées ou un mélange de ces substances et qu'au moins une partie des biomolécules est immobilisée sur la surface du support.

2. Support selon la revendication 1 **caractérisé en ce qu'**un sel dans lequel les biomolécules sont placées, est un sel de la série de Hofmeister.

3. Support selon la revendication 1 ou 2 **caractérisé en ce qu'**un sel dans lequel les biomolécules sont placées, est un chlorure de sodium ou un sulfate d'ammonium.

4. Support selon l'une des revendications 1 à 3 **caractérisé en ce que** la/les substance/s est/sont des sels pour solutions tamponnées pour tampons de phosphate, de citrate, d'acétate, de tris, de triéthanolamine, hepes, pipes ou mops.

5. Support selon l'une des revendications 1 à 4 **caractérisé en ce que** la substance ou le mélange de substances contient en plus au moins un stabilisateur pour biomolécules.

6. Support selon l'une des revendications 1 à 4 **caractérisé en ce que** les biomolécules sont des acides nucléines, des (glyco-) protéines, des (glyco-) peptides, des (glyco-) lipides ou des glucides.

7. Support selon l'une des revendications 1 à 6 **caractérisé en ce que** les biomolécules sont des anticorps, des allergènes, de l'avidine, un CRP, de l'hémoglobine, du hCG, hFabp, IgA, IgE, myoglobine et/ou du neutravidine.

8. Support selon l'une des revendications 1 à 7 **caractérisé en ce que** le support est enfermé au sec pour le stockage et le transport dans une enveloppe pratiquement étanche à la vapeur d'eau.

9. Support selon l'une des revendications 1 à 8 **caractérisé en ce que** le matériau de support est un plastique, un verre, un métal ou une liaison anorganique.

10. Support selon l'une des revendications 1 à 9 **caractérisé en ce que** le support est formé de polyméthacrylate de méthyle, de polymères et copolymères de cyclooléfine, de polyéthylène, de polystyrène ou polycarbonate.

11. Support selon l'une des revendications 1 à 10 **caractérisé en ce que** la surface du support est modifiée par des processus physico-chimiques.

12. Support selon la revendication 11 **caractérisé en ce que** la surface du support présente une tension de surface limite réduite par rapport à l'eau désionisée à la suite de la modification.

13. Support selon la revendication 11 **caractérisé en ce qu'**à la suite de la modification, l'angle de contact d'une goutte d'eau désionisée diminue d'au moins 5° par rapport à une surface non modifiée.

14. Support selon l'une des revendications 1 à 13 **caractérisé en ce que** la partie des cristaux cubiques formés à la surface du support est ≤ 50 % de volume.

15. Procédé d'immobilisation de biomolécules aux surfaces de support pour exécuter des tests d'affinité dans lesquels à la surface du support sont appliquées des biomolécules dans une solution tamponnée qui contient au moins une substance à caractère ionique du groupe des sels, des polyanions, des polycations, des zwitterions, des ampholytes et des sels pour solutions tamponnées, immédiatement après le solvant est enlevé de la solution tamponnée par séchage, les biomolécules étant placées dans la substance ou un mélange de ces substances et au moins une partie des biomolécules étant immobilisée à la surface du support.

16. Procédé selon la revendication 15 **caractérisé en ce qu'**une solution tamponnée est utilisée avec une concentration de sel dans la plage de 100 mM à 1,5 M.

17. Procédé selon la revendication 15 ou 16 **caractérisé en ce qu'**une solution tamponnée biologique contenant en plus des stabilisateurs pour les biomolécules est utilisée.

18. Procédé selon l'une des revendications 15 à 17 **caractérisé en ce qu'**une solution tamponnée avec une valeur pH entre 3 et 10 est utilisée.

19. Procédé selon l'une des revendications 15 à 18 **caractérisé en ce que** la surface respective du support est modifiée avant l'application de la solution biologique contenant des biomolécules est modifiée par des processus thermiques, plasma-chimiques, électrochimiques ou chimiques humides.

20. Procédé selon la revendication 19 **caractérisé en ce que** la modification réduit la tension de surface limite de la surface du support.

21. Procédé selon la revendication 19 ou 20 **caractérisé en ce que** la modification est exécutée avec une décharge en couronne ou de barrière.

22. Procédé selon la revendication 19 **caractérisé en ce qu'**un traitement thermique est exécuté par un réchauffement du support pour passer à une température d'au moins 40°C avec par la suite un refroidissement défini.

23. Procédé selon la revendication 19 **caractérisé en ce qu'**un traitement chimique humide est exécutée avec de l'acide sulfurique contenant du permanganate de potassium.

24. Procédé selon la revendication 19 **caractérisé en ce que** la modification de surface est exécutée par un traitement au plasma gazeux à haute ou basse pression.

25. Procédé selon la revendication 24 **caractérisé en ce que** le plasma gazeux est généré avec de l'oxygène, de l'azote et/ou du dioxyde de carbone.

26. Procédé selon l'une des revendications 15 à 25 **caractérisé en ce que** l'application s'effectue via dispersion, enduction par trempage ou enduction centrifuge.

27. Procédé selon l'une des revendications 15 à 26 **caractérisé en ce que** le séchage permettant d'enlever le solvant s'effectue sous vide et à une température de 50°C dans une période ne dépassant pas env. 10 minutes.

28. Procédé selon l'une des revendications 15 à 27 **caractérisé en ce que** le séchage permettant d'enlever le solvant s'effectue à température de salle et à une humidité de l'air dans une plage entre 35 et 40%.

29. Procédé selon l'une des revendications 15 à 28 **caractérisé en ce** le support est complètement enveloppe pendant le stockage et le transport au sec dans une enveloppe pratiquement étanche à la vapeur d'eau.
